# EUROPEAN PATENT SPECIFICATION

(11) **EP 3 328 208 B1**
(45) Date of publication and mention of the grant of the patent: **20.11.2019**
(21) Application number: 15766613.2
(22) Date of filing: 31.07.2015
(51) Int. Cl.: A23C 9/123, A23C 9/13, C12N 1/20

(54) **METHODS FOR THE PREPARATION OF FERMENTED PRODUCTS COMPRISING BIFIDOBACTERIA**
VERFAHREN ZUR HERSTELLUNG FERMENTIERTER PRODUKTE MIT BIFIDOBAKTERIEN
PROCÉDÉS DE PRÉPARATION DE PRODUITS FERMENTÉS COMPRENANT DES BIFIDOBACTÉRIES

(43) Date of publication of application: 06.06.2018
(73) Proprietor: Compagnie Gervais Danone, 75009 Paris (FR)
(72) Inventor: MARCHAL, Laurent, F-91360 Villemoisson sur Orge (FR); DAVAL, Christophe, 91120 Palaiseau (FR)
(74) Representative: Gevers & Orès
(86) International application number: PCT/IB2015/055827
(87) International publication number: WO 2017/021754

(56) References cited:
- EP-A1- 2 380 446
- WO-A1-2014/096901
- JP-A- 2003 219 861
- PRASANNA P H P ET AL: "Bifidobacteria in milk products: An overview of physiological and biochemical properties, exopolysaccharide production, selection criteria of milk products and health benefits", FOOD RESEARCH INTERNATIONAL, ELSEVIER APPLIED SCIENCE, BARKING, GB, vol. 55, 16 November 2013 (2013-11-16), pages 247-262, XP028669431, ISSN: 0963-9969, DOI: 10.1016/J.FOODRES.2013.11.013
- Anonymous: "MRS-Agar", , 19 June 2012 (2012-06-19), XP002748664, Retrieved from the Internet: URL:http://web.archive.org/web/20120619153 535/http://www.techno-lab.de/mikrobiologie /msr_agar.htm [retrieved on 2015-10-26]
- DATABASE GNPD [Online] MINTEL; Anonymous: "Fermented milk with oats and nuts", XP002748665, Database accession no. 2360579
- DATABASE GNPD [Online] MINTEL; Anonymous: "Fermented Milk with American Blueberry", XP002748666, Database accession no. 2473209
- DATABASE GNPD [Online] MINTEL; Anonymous: "Real Banana Drinking Yoghurt", XP002748667, Database accession no. 3100179
- Esther Sendra Nadal ET AL: "Food Formulation to Increase Probiotic Bacteria Action or Population" In: "Bioactive Foods in Promoting Health", 1 January 2010 (2010-01-01), Elsevier, XP55053097, ISBN: 978-0-12-374938-3 pages 335-351, DOI: 10.1016/B978-0-12-374938-3.00022-0, the whole document
- RACCACH M: "Manganese and lactic acid bacteria", JOURNAL OF FOOD PROTECTION, INTERNATIONAL ASSOCIATION FOR FOOD PROTECTION, US, vol. 48, no. 10, 1 September 1985 (1985-09-01), pages 895-898, XP008110593, ISSN: 0362-028X

## Description

### FIELD OF THE INVENTION

The present invention provides methods for the preparation of fermented food products comprising *Bifidobacterium* species, namely *Bifidobacterium animalis* subsp. *animalis* and/or *Bifidobacterium breve.* The present invention further provides products prepared by such methods as well as inoculums of use in such methods.

### BACKGROUND

According to a definition approved by a joint Food and Agriculture Organization of the United Nations/World Health Organization (FAO/WHO) expert Consultation on Health and Nutritional properties of powder milk with live lactic acid bacteria in 2001, probiotics are "live microorganisms which when administered in adequate amounts confer a health benefit on the host". Probiotics are widely available in the form of food products and supplements, including fermented dairy products. The most common dairy probiotics are of the *Lactobacillus* or *Bifidobacterium* genera, although probiotics of the *Escherichia, Enterococcus, Bacillus, Propionibacterium* and *Saccharomyces* genera have also been identified.

*Bifidobacteria* are naturally present in the intestinal microbiota and are amongst the most popular and well characterized probiotic species. The main species present in the human colon are *Bifidobacterium adolescentis, Bifidobacterium bifidum, Bifidobacterium longum ssp infantis, Bifidobacterium breve, Bifidobacterium longum.* Bacteria of the genus *Bifidobacteria* are widely used in commercially available probiotic food products, such as fermented dairy products including cheese, yogurt, frozen yogurt and fermented milk.

As defined in the above-mentioned FAO/WHO guidelines probiotics are live bacteria. Furthermore, a daily intake of at least 10⁸ to 10⁹ viable cells has been recommended as the minimum dose to provide a beneficial effect (Silva et al, J. Appl. Microbiol. 97 (2004) 29-37). Thus not only is it necessary to ensure that sufficient probiotic counts are established during preparation of the product, they must also be maintained for the shelf-life of the product. These requirements pose challenges to the manufacture of *Bifidobacteria* probiotic dairy products as *Bifidobacteria* have poor proteolytic activity and grow slowly in milk. Furthermore, *Bifidobacteria* viability in fermented dairy products is limited by their sensitivity to acidic pH. This is particularly of relevance to yogurt-type products as acidity may increase in such products during storage, a phenomenon termed "post-acidifcation"; which may consequently affect *Bifidobacteria* viability. *Bifidobacteria* survival during storage is also affected by other factors such as temperature and oxygen levels, as well as lactic and acetic acid content. Finally, *Bifidobacteria* growth has shown to be inhibited by co-culture with typical starter cultures used in the preparation of fermented dairy-products such as yogurts.

The use of relatively high quantities of *Bifidobacteria* in the inoculum as well as growth enhancers may be used to aid the expansion and maintenance of the probiotic populations. Growth enhancers known in the art include the use of yeast extract, casein hydrolysates, amino acids as well as other micronutrients and trace elements. Cysteine has been disclosed as an enhancer of growth and maintenance of *Bifidobacterium* populations in patent application PCT/FR2006/001688, whereas manganese has been proposed as a growth stimulant for *Bifidobacterium animalis subsp. lactis* strain CHCC5445 in patent application EP 2380446. In this EP application it was shown that manganese increases the acidification/growth of *Bifidobacterium animalis subsp. lactis* strain CHCC5445 in milk but only when the milk is supplemented with a suitable amount of peptides.

### SUMMARY OF THE INVENTION

The subject matter of the invention is defined by claims 1 to 15.

The present invention provides a process for the preparation of a fermented milk product comprising the fermentation of a mixture comprising milk, *Bifidobacterium animalis subsp. animalis* and/or *Bifidobacterium breve* and a manganese compound. Surprisingly it is herein disclosed that the addition of a manganese compound is able to enhance the growth of said *Bifidobacterium* subspecies in milk without the requirement for peptide supplementation.

The invention further provides products obtainable by means of such processes as well as uses of manganese compounds in the culture of *Bifidobacterium animalis subsp. animalis* and/or *Bifidobacterium breve.*

In an additional embodiment the present invention provides an inoculum for the preparation of fermented milk products comprising a manganese compound and *Bifidobacterium selected from Bifidobacterium animalis subsp. animalis* and/or *Bifidobacterium breve.*

### DETAILED DESCRIPTION

As used herein the term "CNCM I-" followed by a 4 digit number shall be taken to refer to a strain deposited at the Collection Nationale de Culture de Microorganismes (CNCM, Paris, France) under the Budapest Treaty with an accession number corresponding to said 4 digit number, e.g. CNCM I-4602 or CNCM I-4321.

As used herein the term "stable composition" shall be taken to mean a composition that does not present sedimentation and/or serum separation.

As used herein the term "x% (w/w)" is equivalent to "x g per 100 g".

As used herein the term "fermented milk" shall be taken to mean a product or composition derived from milk by the acidifying action of at least one lactic acid bacterium.

As used herein the term "spoonable" shall be taken to mean a solid or semi-solid that may be consumed by means of a spoon or other utensil.

As used herein the term "fermentation" shall be taken to mean the metabolism of a substance by bacteria, yeasts, or other microorganisms.

As used herein the term "cfu" or "CFU" shall be taken to be an abbreviation of the term "colony forming unit".

As used herein the term "in the free form" shall be taken to mean that which is not bound to other amino acids by a peptide bond within peptides, polypeptides or proteins.

As used herein reference to a bacterial strain or species shall be taken to include functionally equivalent bacteria derived therefrom such as but not limited to mutants, variants or genetically transformed bacteria. These mutants or genetically transformed strains can be strains wherein one or more endogenous gene(s) of the parent strain has (have) been mutated, for instance to modify some of their metabolic properties (*e.g.*, their ability to ferment sugars, their resistance to acidity, their survival to transport in the gastrointestinal tract, their post-acidification properties or their metabolite production). They can also be strains resulting from the genetic transformation of the parent strain to add one or more gene(s) of interest, for instance in order to give to said genetically transformed strains additional physiological features, or to allow them to express proteins of therapeutic or prophylactic interest that one wishes to administer through said strains. These mutants or genetically transformed strains can be obtained from the parent strain by means of conventional techniques for random or site-directed mutagenesis and genetic transformation of bacteria, or by means of the technique known as "genome shuffling". In the present text, strains, mutants and variants derived from a parent species or strain will be considered as being encompassed by reference to said parent species or strain, e.g. the phrases *"Bifidobacterium animalis subsp. animalis"* and "strain CNCM I-4602" shall be taken to include strains, mutants and variants derived therefrom.

As used herein the term "yeast extract" shall be taken to mean concentrates of soluble compounds of yeast cells. In this regard reference may be made in particular to the article "Yeast extracts: production, properties and components" by Rolf Sommer (9th International Symposium on Yeasts), from which the information below is extracted.
Yeast extracts are mainly produced by autolysis, i.e. cell hydrolysis is carried out without the addition of other enzymes. Yeast extract or yeast autolysate are used mainly in the fermentation industry and in the agri-food industry. The main raw material used in order to produce the yeast extract is constituted by yeasts with a high concentration of proteins (strains of *Saccharomyces cerevisiae*) cultured on media based on molasses or is constituted by yeasts from debittered beer (strains of *Saccharomyces cerevisiae* or *Saccharomyces uvarum*)*.* Other raw materials used are yeasts such as *Kluyveromyces fragilis* (fermented on lactoserum) or *Candida utilis* (cultured on carbohydrate-rich waste originating from of the timber industry or on ethanol) or also special strains of baker's yeasts, in order to produce yeast extract containing 5'-nucleotides.
Methods for the preparation of yeast extract are known in the art, autolysis is the dissociation process most frequently used production process. During this process, the yeasts are degraded by their endogenous enzymes. The autolysis process can be initiated by osmotic shock or controlled temperature, causing cell death without inactivating the endogenous enzymes (in particular the proteases). The temperature, controlled pH and the duration of the autolysis are decisive factors in a standardized autolysis process. By adding salts or enzymes (for example proteases or mixtures of proteases and peptidases) relative to the "standard" autolysis, the protein degradation of the yeast cells can be controlled.

As an alternative to autolysis, the yeast extract can be produced by thermolysis (for example by boiling the yeasts in water at 100° C.), plasmolysis (treatment with strong saline solutions at a temperature below 100° C.) and mechanical degradation (high-pressure homogenization or grinding). Soluble compounds are separated from the insoluble cell walls and concentrated with an evaporator with stirring or falling film evaporator, followed by optional stages of filtration, concentration under partial vacuum and rapid sterilization.

### Processes of the invention

The present description provides a process for the preparation of a fermented milk product comprising the following steps:
i) providing a mixture comprising:
   a) milk
   b) at least one bacteria species, comprising at least one *Bifidobacteria* selected from the group consisting of *Bifidobacterium animalis subsp. animalis* and *Bifidobacterium breve,*
   c) a manganese compound
ii) fermentation of said mixture to provide a fermented milk product.

The present invention relates to a process for the preparation of a fermented milk product comprising the following steps:
i) providing a mixture comprising:
   a) milk
   b) at least one bacteria species, comprising at least one Bifidobacteria selected from the group consisting of *Bifidobacterium animalis* subsp. *animalis* and *Bifidobacterium breve,*
   c) a manganese compound
ii) fermentation of said mixture to provide a fermented milk product,
wherein the amount of manganese compound in said mixture i) is at least 5 mg/l.It is to be understood that the manganese compound of ii) refers to manganese that is present outside the *Bifidobacteria* cell, *i.e.,* is not included within the *Bifidobacteria* cell.

Preferably the mixture comprises at least about 30 % (w/w) milk, more preferably at least about 50% (w/w) milk and even more preferably at least about 70% (w/w) milk. Preferably said milk is vegetal and/or animal milk, more preferably soya, almond, oat, hemp, coconut, rice, goat, ewe, camel, mare or cow milk, and most preferably to cow milk. Preferably said milk(s) are heat-treated, typically pasteurized, to ensure sterility. Preferably said heat treatment is carried out prior to the preparation of the fermented milk product.

Preferably said mixture comprises one or more of skimmed, partially-skimmed or non-skimmed milk. Preferably said milk or milks may be in liquid, powdered and/or concentrated form. In one embodiment said mixture further comprises cream. In one embodiment said mixture further comprises plant and/or fruit juices. In one embodiment said milk or milks may be enriched or fortified with further milk components or other nutrients such as but not limited to vitamins, minerals, trace elements or other micronutrients.

The manganese compound may be any such compound suitable for animal or human consumption and utilizable by Bifidobacteria. Typically the manganese compound may be a manganese salt or solution. It is particularly preferred that the manganese compound is a manganese salt such as an acetate, carbonate, chloride, soluble citrate, gluconate, orthophosphate, dibasic phosphate, sulfate or oxide. It is particularly preferred that the manganese compound is MnCl₂ and/or MnSO₄.

In an embodiment, the invention relates to a process as defined above, wherein the amount of manganese compound in said mixture i) is between 5-100 mg/l, preferably between 5-65 mg/l. Preferably the amount of manganese compound in said mixture is preferably at least 10 mg/l, 15 mg/l, 20 mg/l, 25 mg/l, 30 mg/l, 35 mg/l, 40 mg/l, 45 mg/l, 50 mg/l, 55 mg/l, 60 mg/l, 65 mg/l or more.

Preferably the amount of *Bifidobacteria* in said mixture i) is 10⁶-10⁸ cfu/g , more preferably 10⁶-10⁷ cfu/g. In one embodiment the *Bifidobacterium* is *Bifidobacterium animalis subsp. animalis,* preferably strain CNCM I-4602. In an alternative embodiment the *Bifidobacterium* is *Bifidobacterium Breve,* preferably strain CNCM I-4321.

In an embodiment, the invention relates to a process as defined above, wherein said *Bifidobacteria* is *Bifidobacterium animalis* subsp. *animalis* strain CNCM I-4602 and/or *Bifidobacterium breve* strain CNCM 1-4321.

It is particularly preferred that the fermented milk according to the invention is obtained by the acidifying action of at least one, two, three, four, five, six, seven or more lactic acid bacteria strains. Accordingly in one embodiment the mixture of i) further comprises at least one, two, three, four, five, six, seven or more lactic acid bacteria strains.

The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckei,* in particular *L. delbruckei supsb. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*)*; Streptococci* (for example *Streptococcus thermophilus*)*; Lactococci* (for example *Lactococcus lactis,* typically *Lactococcus lactis subsp. lactis* or *Lactococcus lactis subsp. cremoris*)*.* Typically a mixture or association of a plurality of species of lactic acid bacteria may be used, typically a mixture or association of Lactobacillus and Streptococcus. For the preparation of yogurt this typically includes *Lactobacillus bulgaricus* (also referred to as *Lactobacillus delbruckei subsp. bulgaricus*) and *Streptococcus thermophilus,* optionally with additional microorganisms such as but not limited to probiotic species or other species that may provide desirable organoleptic or other qualities to the composition, e.g. *Lactococcus lactis.*

In a further embodiment the lactic acid bacteria may further comprise a probiotic bacteria. Probiotic bacteria are known by the one skilled in the art. Examples of probiotic bacteria include some *Bifidobacteria* and *Lactobacilli,* such as *Bifidobacterium brevis, Bifidobacterium animalis, Bifidobacterium animalis subsp. lactis, Bifidobacterium infantis, Bifidobacterium longum, Lactobacillus helveticus, Lactobacillus casei, Lactobacillus easel paracasei, Lactobacillus acidophilus, Lactobacillus rhamnosus, Lactobacillus plantarum, Lactobacillus reuteri, Lactobacillus delbrueckii subsp bulgaricus, Lactobacillus delbrueckii subsp lactis, Lactobacillus delbrueckii subsp delbrueckii, Lactobacillus brevis* and *Lactobacillus fermentum.*

Accordingly in one embodiment, the mixture of i) further comprises a plurality of species of lactic acid bacteria comprising either, or more preferably both, *Streptococcus thermophilus* and/or *Lactobacillus delbrueckii subsp. bulgaricus.* In a further embodiment, the mixture of i) further comprises a plurality of species of lactic acid bacteria comprising *Streptococcus thermophilus, Lactobacillus delbrueckii* subsp. *bulgaricus* and *Lactococcus lactis.*

Preferably the total amount of lactic acid bacteria in said mixture i) is about 10⁶-10⁸ cfu/g, more preferably about 10⁶-10⁷ cfu/g. Preferably the amount of Lactobacillus bulgaricus in said mixture i) is about 10⁶-10⁸ cfu/g, more preferably about 10⁶-10⁷ cfu/g. Preferably the amount of Streptococcus thermophilus in said mixture i) is about 10⁶-10⁸ cfu/g, more preferably about 10⁶-10⁷ cfu/g.

Methods for the preparation of fermented milk products, such as yogurts or equivalents thereof, are well-known in the art. Typically a fermented milk product is prepared by culture of milks at a suitable temperature with suitable microorganisms to provide a reduction in pH, preferably to a pH equal to or lower than 5, preferably between about 3 and 4.5; more preferably between about 3.5 and about 4.5. The pH can be adjusted by controlling the fermentation by the microorganism and stopping it when appropriate, for example by cooling.

Suitable temperatures for milk fermentation are typically about 36°C to about 44°C and the temperature is maintained for an incubation time sufficient to provide the desired reduction in pH.

For the preparation of a fermented milk product the temperature at the start of fermentation is typically about 36°C to about 43°C, in particular about 37°C to about 40°C, the temperature at the end of fermentation is typically about 37°C to about 44°C, in particular about 38°C to about 41°C. The fermentation time is typically about 6 to about 11 hours.

Subsequent to the fermentation the fermented milk is cooled. Optionally a stage of intermediate cooling of the fermented milk may be performed to provide a pre-cooled fermented milk having a temperature of between about 22°C and about 4°C. Typically the intermediate cooling time is about 1 hour to about 4 hours, in particular about 1 hour 30 minutes to about 2 hours. The pre-cooled fermented milk is typically stored for up to 40 hours or less.

Preferably a stage of final cooling of the fermented milk is performed such that the temperature at the start of the final cooling is less than about 22°C and the temperature at the end of the final cooling is about 4°C to about 10°C. The cooled product may then be stored, transported and/or distributed at a temperature from about 1°C to about 10°C for at least about 30 days, at least about 60 days or at least about 90 days.

According to a further embodiment, the process for the preparation of a fermented milk product as defined above optionally comprises a stage of stirring at a pressure of at least 20 bars, or performing a dynamic smoothing, to obtain a composition having the desired viscosity, typically a viscosity of up to 20 mPa.s. Stirring or dynamic smoothing operations provide some shear to composition that typically allow a viscosity drop. Such operations are known by the one skilled in the art, and can be operated with conventional appropriate equipment. This stage is typically performed at cold temperature, for example at a temperature of form 1°C to 20°C.

Without intending to be bound to any theory, it is believed that applying some shear at cold temperature, typically by stirring at high pressure or by performing a dynamic smoothing, can lead to a fluid gel formation within the composition, that provides improved stability even at a low viscosity of up to 20 mPa.s.

According to a further embodiment, the process for the preparation of a fermented milk product as defined above optionally comprises a stage of addition of an intermediate preparation prior or subsequent to fermentation step ii), said intermediate preparation comprising a preparation of fruits and/or cereals and/or additives such as flavorings and/or colorings. Said intermediate preparation can in particular contain thickeners (soluble and insoluble fibres, alginates, carragheenans, xanthan gum, pectin, starch, in particular gelatinized, gelan gum, cellulose and its derivatives, guar and carob gum, inulin) or sweeteners (aspartame, acesulphame K, saccharine, sucralose, cyclamate) or preservatives. Examples of flavorings include but are not limited to apple, orange, strawberry, kiwi fruit, cocoa flavoring etc. Examples of colorings include but are not limited to beta-carotene, carmine, cochineal red. Moreover, the preparation of the abovementioned fruits can comprise fruits which are whole or in pieces or in jelly or in jam, making it possible for example to obtain fruit yogurts. The intermediate preparation can also contain plant extracts (soya, rice etc.).

Preferably the fermented milk product, according to the invention comprises up to about 30% (w/w) of said intermediate preparation, e.g. up to about 10%, 15%, 20%, 25% (w/w).

The product is optionally packaged in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight. Sealing can be performed for example with a cap or with a lid. The product may be packaged either prior to or subsequent to the fermentation step ii). Accordingly in one embodiment the invention provides a process of the invention wherein the mixture i) is packaged prior to the fermentation of step ii). In an alternative embodiment the fermented milk of step ii) is packaged.

In one embodiment the invention provides a process of the invention wherein the mixture i) further comprises sulphur-containing amino acid and/or yeast extract.

The sulphur-containing amino acid is preferably cysteine (L-cysteine) or methionine as well as their derivatives, optionally in the form of a salt. Typically the sulphur-containing amino acid may be monohydrated L-cysteine hydrochloride (monohydrated (R)-2-amino-mercaptopropionic acid monohydrochloride) or L-methionine ((S)-2-amino-4-methylthio-butyric acid). Preferably, the sulphur-containing amino acids according to the invention are used in reduced form, i.e. the sulphhydryl group --SH is reduced. This preferred form of the sulphur-containing amino acids therefore excludes in particular cystine, the oxidized form of cysteine involving the combination of two cysteines via a disulphide bridge.

Yeast extract may be in the form of liquid yeast extract (dried matter: 50 to 65%); viscous paste-type yeast extract (dried matter: 70 to 80%); dry yeast extract powder. Typically yeast extract suitable for use in fermentation processes may have a protein content of 75%-75%, sodium content less than 0.5%, polysaccharide content less than 5%, oligosaccharide content less than 1% and lipid content less than 0.5%. Typically the protein content of yeast extract suitable for use in fermentation may be about 35-40% free amino acids; 10-15% di, tri and tetrapeptides (MW < 600 Da), 40-45% oligopeptides (MW of 2000-3000 Da); 2-5% oligopeptides (MW of 3000-100000 Da).

Preferably the amount of cysteine in said mixture i) is between about 1-100 mg/l, more preferably 5-75 mg/l. Preferably the amount of yeast extract in said mixture i) is up to about to 0.5%.

The invention also relates to the use of a manganese compound in the culture of *Bifidobacterium animalis* subsp. *animalis* and/or *Bifidobacterium breve,* wherein the amount of manganese compound is at least 50 mg/l.

The present description provides the use of a manganese compound in the culture of at least one *Bifidobacteria* species, comprising at least one *Bifidobacterium* selected from the group consisting of *Bifidobacterium animalis subsp. animalis* and *Bifidobacterium breve.* Typically the amount of manganese compound used is between 5-100 mg/l of culture medium, more preferably between about 5-65 mg/l , accordingly the amount of manganese compound used is preferably at least 1 mg/l, 5 mg/l, 10 mg/l, 15 mg/l, 20 mg/l, 25 mg/l, 30 mg/l, 35 mg/l, 40 mg/l, 45 mg/l, 50 mg/l, 55 mg/l, 60 mg/l, 65 mg/l or more.

It is particularly preferred that the manganese compound is a manganese salt typically MnCl₂ and/or MnSO₄. Preferably the amount of *Bifidobacteria* used is about 10⁶-10⁸ cfu/g of culture medium, more preferably 10⁶-10⁷ cfu/g . In one embodiment the *Bifidobacteria* is *Bifidobacterium animalis subsp. animalis,* preferably strain CNCM I-4602. In an alternative embodiment the *Bifidobacteria* is *Bifidobacterium Breve,* preferably strain CNCM I-4321.

In an embodiment, the invention relates to the use as defined above, wherein said *Bifidobacteria* is *Bifidobacterium animalis* subsp. *animalis* strain CNCM I-4602 and/or *Bifidobacterium* breve *strain* CNCM 1-4321.

### Inoculum of the invention

The invention also relates to an inoculum for the preparation of fermented milk products comprising
i) at least one bacteria species, comprising at least one Bifidobacteria selected from the group consisting of *Bifidobacterium animalis* subsp. *animalis* and *Bifidobacterium breve* and,
ii) a manganese compound in an amount of at least 0.1% w/w
wherein i) and ii) are provided in the form of a mixture or as a kit of parts.

The present description provides an inoculum for the preparation of fermented milk products comprising:
i) at least one bacteria species, comprising at least one *Bifidobacteria* selected from the group consisting of *Bifidobacterium animalis subsp. animalis* and *Bifidobacterium breve* and,
ii) a manganese compound.

It is to be understood that the manganese compound of ii) refers to manganese that is present outside the *Bifidobacteria* cell, *i.e.,* is not included within the *Bifidobacteria* cell.

The inoculum of the invention is suitable for the direct inoculation of at least one *Bifidobacteria* strain into milk to provide fermented milk products of the invention.

In one embodiment i) and ii) may be provided as a mixture. In an alternative embodiment i) and ii) are provided as a kit of parts.

The inoculum or bacteria thereof are preferably fresh, frozen or lyophilized. The inoculum may be in liquid, dry, spray-dried or solid form. It is particularly preferred that the inoculum is in liquid form. Typically the inoculum is provided to the dairy mix in quantities of up to about 500 mg/l.

The manganese compound may be any such compound suitable for animal or human consumption and utilizable by Bifidobacteria. Typically the manganese compound may be a manganese salt or solution. It is particularly preferred that the manganese compound is a manganese salt such as an acetate, carbonate, chloride, soluble citrate, gluconate, orthophosphate, dibasic phosphate, sulfate or oxide. It is particularly preferred that the manganese compound is MnCl₂ and/or MnSO₄.

Preferably the amount of manganese compound in said inoculum i) is about preferably at least 0.1%, 1%, 5%, 10% or more (w/w). Typically between 0.1 and 20% (w/w), more preferably between about 1 and 13% (w/w).

Preferably the amount of *Bifidobacteria* in said inoculum i) is about10¹⁰-10¹¹ cfu/g or about10¹⁰-10¹¹ cfu/ml when the inoculum is provided in liquid form. In one embodiment the *Bifidobacteria* is *Bifidobacterium animalis subsp. animalis,* preferably strain CNCM I-4602. In an alternative embodiment the *Bifidobacteria* is *Bifidobacterium Breve,* preferably strain CNCM I-4321.

In an embodiment, the invention relates to an inoculum as defined above, wherein said *Bifidobacteria* is *Bifidobacterium animalis* subsp. *animalis* strain CNCM I-4602 and/or *Bifidobacterium breve* strain CNCM 1-4321.

In a further embodiment the inoculum further comprises at least one, two, three, four, five, six, seven or more lactic acid bacteria strains. The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckei,* in particular *L. delbruckei supsb. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*)*; Streptococci* (for example *Streptococcus thermophilus*)*; Lactococci* (for example *Lactococcus lactis,* typically *Lactococcus lactis subsp. lactis* or *Lactococcus lactis subsp. cremoris*)*.* Typically a mixture or association of a plurality of species of lactic acid bacteria may be used, typically a mixture or association of Lactobacillus and Streptococcus. For the preparation of yogurt this typically includes *Lactobacillus bulgaricus* (also referred to as *Lactobacillus delbruckei subsp. bulgaricus*) and *Streptococcus thermophilus,* optionally with additional microorganisms such as but not limited to probiotic species or other species that may provide desirable organoleptic or other qualities to the composition, e.g. *Lactococcus lactis.*

In a preferred embodiment, the inoculum further comprises a plurality of species of lactic acid bacteria comprising either, or more preferably both, *Streptococcus thermophilus* and/or *Lactobacillus delbrueckii* subsp. *bulgaricus.* In an alternative embodiment, the inoculum further comprises a plurality of species of lactic acid bacteria comprising *Streptococcus thermophilus, Lactobacillus delbrueckii* subsp. *bulgaricus* and *Lactococcus lactis.*

In a further embodiment the invention provides an inoculum further comprising iii) sulphur-containing amino acid and/or yeast extract.

The sulphur-containing amino acid is preferably cysteine (L-cysteine) or methionine as well as their derivatives, optionally in the form of a salt. Typically the sulphur-containing amino acid may be monohydrated L-cysteine hydrochloride (monohydrated (R)-2-amino-mercaptopropionic acid monohydrochloride) or L-methionine ((S)-2-amino-4-methylthio-butyric acid). Preferably, the sulphur-containing amino acids according to the invention are used in reduced form, i.e. the sulphhydryl group --SH is reduced. This preferred form of the sulphur-containing amino acids therefore excludes in particular cystine, the oxidized form of cysteine involving the combination of two cysteines via a disulphide bridge.

Yeast extract may be in the form of liquid yeast extract (dried matter: 50 to 65%); viscous paste-type yeast extract (dried matter: 70 to 80%); dry yeast extract powder. Typically yeast extract suitable for use in fermentation processes may have a protein content of 75%-75%, sodium content less than 0.5%, polysaccharide content less than 5%, oligosaccharide content less than 1% and lipid content less than 0.5%. Typically the protein content of yeast extract suitable for use in fermentation may be about 35-40% free amino acids; 10-15% di, tri and tetrapeptides (MW < 600 Da), 40-45% oligopeptides (MW of 2000-3000 Da); 2-5% oligopeptides (MW of 3000-100000 Da).

### Kits

The present description provides a kit (i.e., article of manufacture) for the preparation of fermented milk products comprising of:
i) A receptacle containing at least one bacteria species, comprising at least one *Bifidobacteria* selected from the group consisting of *Bifidobacterium animalis subsp. animalis* and *Bifidobacterium breve* and,
ii) A receptacle containing a manganese compound.

The kit optionally further comprises cysteine, yeast extract and or lactic acid bacteria.

Accordingly the kit optionally further comprises iiia) A receptacle containing sulphur-containing amino acid. The kit optionally comprises iiib) A receptacle containing yeast extract.
The sulphur-containing amino acid is preferably cysteine (L-cysteine) or methionine as well as their derivatives, optionally in the form of a salt. Typically the sulphur-containing amino acid may be monohydrated L-cysteine hydrochloride (monohydrated (R)-2-amino-mercaptopropionic acid monohydrochloride) or L-methionine ((S)-2-amino-4-methylthio-butyric acid). Preferably, the sulphur-containing amino acids according to the invention are used in reduced form, i.e. the sulphhydryl group --SH is reduced. This preferred form of the sulphur-containing amino acids therefore excludes in particular cystine, the oxidized form of cysteine involving the combination of two cysteines via a disulphide bridge.

Yeast extract may be in the form of liquid yeast extract (dried matter: 50 to 65%); viscous paste-type yeast extract (dried matter: 70 to 80%); dry yeast extract powder. Typically yeast extract suitable for use in fermentation processes may have a protein content of 75%-75%, sodium content less than 0.5%, polysaccharide content less than 5%, oligosaccharide content less than 1% and lipid content less than 0.5%. Typically the protein content of yeast extract suitable for use in fermentation may be about 35-40% free amino acids; 10-15% di, tri and tetrapeptides (MW < 600 Da), 40-45% oligopeptides (MW of 2000-3000 Da); 2-5% oligopeptides (MW of 3000-100000 Da).

The kit optionally further comprises iiic) A receptacle containing of at least one, two, three, four, five, six, seven or more lactic acid bacteria strains. The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckei,* in particular *L. delbruckei supsb. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*)*; Streptococci* (for example *Streptococcus thermophilus*)*; Lactococci* (for example *Lactococcus lactis,* typically *Lactococcus lactis subsp. lactis* or *Lactococcus lactis subsp. cremoris*)*.* Typically a mixture or association of a plurality of species of lactic acid bacteria may be used, typically a mixture or association of Lactobacillus and Streptococcus. For the preparation of yogurt this typically includes *Lactobacillus bulgaricus* (also referred to as *Lactobacillus delbruckei subsp. bulgaricus*) and *Streptococcus thermophilus,* optionally with additional microorganisms such as but not limited to probiotic species or other species that may provide desirable organoleptic or other qualities to the composition, e.g. *Lactococcus lactis.*

In a preferred embodiment, said lactic acid bacteria comprise either, or more preferably both, *Streptococcus thermophilus* and/or *Lactobacillus delbrueckii* subsp. *bulgaricus.* In an alternative embodiment, said lactic acid bacteria comprises a plurality of species of lactic acid bacteria comprising *Streptococcus thermophilus, Lactobacillus delbrueckii* subsp. *bulgaricus* and *Lactococcus.*

Optionally the kit of parts may further comprise instructions for use of i) and ii) for the preparation of fermented milk products.

### Fermented milk products of the invention

In a further embodiment the present invention provides fermented milk products obtainable by means of a process of the invention or prepared by means of an inoculum of the invention. The product according to the invention is suitable for consumption or ingestion, preferably by oral means. Accordingly the product comprises or consists of comestible matter. It is particularly preferred that the products of the invention are substantially free of pathogenic or toxicogenic matter. The product according to the invention may be a pharmaceutical product, a nutraceutical product, and/or a food product.

Preferably the fermented milk product comprises, comprises essentially of or consists of milk that has been subjected to heat treatment at least equivalent to pasteurization, preferably said heat treatment is carried out prior to the preparation of the fermented milk product.

Typically, the milk is pasteurized by means of the following successive steps:
1) a stage of standardization of fatty substances of the raw material so as to obtain a standardized substance,
2) a stage of enrichment with dried matter of the standardized substance obtained in the preceding stage, so as to obtain an enriched substance,
3) a stage of preheating of the enriched substance obtained in the preceding stage, so as to obtain a starting substance,
4) a stage of pasteurization and holding of the starting substance obtained in the preceding stage, so as to obtain a pasteurized and held substance,
5) an optional stage of homogenization of the pasteurized and held substance obtained in the preceding stage, so as to obtain a pasteurized, held and optionally homogenized substance,
6) a stage of initial cooling of the pasteurized, held and optionally homogenized substance obtained in the preceding stage, so as to obtain a pasteurized starting substance that has been held, optionally homogenized, and cooled down.

As used herein "standardization of fatty substances" is taken to mean a stage of bringing the quantity of fats present in the starting substance to a pre-determined level. Enrichment with dried matter involves the addition of proteins and fatty substance in order to modify curd firmness.

As used herein "Holding" is taken to mean a rapid thermization of the milk and makes it possible to destroy the vegetative microbial flora, including pathogenic forms. Its typical duration is from 4 to 10 minutes, in particular from 5 to 8 minutes, and in particular approximately 6 minutes.

As used herein "homogenization" is taken to mean the dispersion of the fatty substances in the milk-type substance into small fat globules. The homogenization is carried out for example at a pressure of 100 to 280 bars, in particular 100 to 250 bars, in particular 100 to 200 bars, in particular approximately 200 bars. This homogenization stage is purely optional. It is in particular absent from the production process of products with 0% fatty substances.

Preferably the fermented milk product comprises above about 0.3 g per 100 g by weight free lactic acid, more preferably above about 0.7 g or 0.6 g per 100 g by weight free lactic acid. Preferably the fermented milk product comprises a protein content at least equivalent to that of the milk or milks from which it is derived, preferably at least about 2.5%, more preferably at least about 3% or 3.5% (w/w). Preferably the fermented milk product has a pH equal to or lower than 5, preferably between about 3 and about 4.5 and more preferably between about 3.5 and about 4.5.

Preferably the fermented milk product has a viscosity lower than 200 mPa.s, more preferably lower than 100 mPa.s and most preferably lower that 60 mPa.s, at 10°C, at a shear rate of 64 s⁻¹. The fermented milk product according to the invention is preferably a product selected from the group comprising yogurt, set yogurt, stirred yogurt, pourable yogurt, yogurt drink, frozen yogurt, kefir, buttermilk, quark, sour cream, fresh cheese and cheese. In one embodiment the fermented milk product according to the invention is a drinkable fermented milk product, more preferably a fermented milk drink such as but not limited to a yogurt drink, kefir etc.. In an alternative embodiment the milk product according to the invention is a fermented milk product that is spoonable such as a set or stirred yogurt or equivalent thereof.

Preferably the fermented milk product, according to the invention, may be stored, transported and/or distributed at a temperature of from 1°C to 10°C for at least about 30 days, at least about 60 days or at least about 90 days from packaging and remain suitable for consumption.

Preferably, the fermented milk product comprises at least 10⁶, more preferably at least 10⁷ and most preferably at least 10⁸ colony forming unit (CFU) of *Bifidobacteria* bacteria according to the invention per gram (g) of product according to the invention. Preferably also the product according to the invention comprises at least about 10¹¹, more preferably at least 10¹⁰ and most preferably at least 10⁹ colony forming unit (CFU) of *Bifidobacteria* bacteria according to the invention per gram (g) of product according to the invention. In one embodiment the *Bifidobacteria* is *Bifidobacterium animalis subsp. animalis,* preferably strain CNCM I-4602. In an alternative embodiment the *Bifidobacteria* is *Bifidobacterium Breve,* preferably strain CNCM I-4321.

In an embodiment, the invention relates to a product as defined above, wherein said *Bifidobacteria* is *Bifidobacterium animalis* subsp. *animalis* strain CNCM I-4602 and/or *Bifidobacterium breve* strain CNCM 1-4321.

Preferably, the fermented milk product is a packaged product that comprises at least 10⁶, more preferably at least 10⁷ and most preferably at least 10⁸ colony forming unit (CFU) of *Bifidobacteria* bacteria according to the invention per gram (g) of product according to the invention subsequent to storage, transport and/or distribution at a temperature of from 1°C to 10°C for at least about 30 days, at least about 60 days or at least about 90 days from packaging.

According to a further embodiment, the fermented milk product further comprises an intermediate preparation comprising a preparation of fruits and/or cereals and/or additives such as flavorings and/or colorings. Said intermediate preparation can in particular contain thickeners (soluble and insoluble fibres, alginates, carragheenans, xanthan gum, pectin, starch, in particular gelatinized, gelan gum, cellulose and its derivatives, guar and carob gum, inulin) or sweeteners (aspartame, acesulphame K, saccharine, sucralose, cyclamate) or preservatives. Examples of flavorings are: apple, orange, strawberry, kiwi fruit, cocoa flavoring etc. Examples of colorings are: beta-carotene, carmine, cochineal red. Moreover, the preparation of the abovementioned fruits can comprise fruits which are whole or in pieces or in jelly or in jam, making it possible for example to obtain fruit yogurts. Preferably the fermented milk product according to the invention comprises up to about 30% (w/w) of said intermediate preparation, e.g. up to about 10%, 15%, 20%, 25% (w/w).

Preferably the fermented milk product, according to the invention is provided in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight.

### EXAMPLES

The aim of the present experiments was to determine the effect of manganese supplementation on milk fermentation by Bifidobacterium.

### Materials and methods

Reconstituted milk was prepared by mixing 112 g skimmed milk powder (Aria Foods) per litre permuted water. Manganese(II) sulfate monohydrate (MnSO4) and L-Cysteine hydrochloride monohydrate were supplied by Sigma-Aldrich. Yeast extract was supplied by AES laboratoire. Milk media was autoclaved at 115°C for 15 minutes prior to inoculation.

Bifidobacteria strains were provided frozen. *Bifidobacterium animalis subsp. animalis* CNCM I-4602 *& Bifidobacterium breve* CNCM I-4321 were supplied by Danone. *Bifidobacterium animalis subsp. lactis* "BB12" was supplied by Chr. Hansen. Strains were inoculated in reconstituted milk media after defrosting at 0.01 % v/v (about 5 x 10⁶ cfu/ml). For *Bifidobacterium animalis subsp. animalis* cultures the reconstituted milk media was supplemented with 2 g/l dextrose, in order to ensure comparable acidification times to *Bifidobacterium breve* and BB12.

Fermentation was carried out at 37 °C and monitored using a pH probe.

### Example 1: Manganese supplementation of reconstituted milk media.

Reconstituted milk was prepared and fermented using *B. animalis, B. breve* or BB12 as provided above. It was observed that the addition of MnSO₄ to the reconstituted milk reduced the time to reach pH 5.5 for B. *animalis animalis* & *B. breve* but not for BB12. At levels of 5mg/L and 65mg/L a 7% reduction was observed in time to pH 5.5 for B. *animalis* subsp. *animalis,* conversely for BB12 an increase in time to pH 5.5 of over 10% was observed at both levels of manganese supplementation. For B. *breve* manganese supplementation was tested only at the level of 65mg/L was carried out, providing a slight reduction in time to pH 5.5.

**Table 1: Addition of MnSO₄ reduces time to pH 5.5 for selected Bifidobacteria**

| Strain | Reconstituted milk | Reconstituted milk + 5mg/L Mn | Reconstituted milk + 65mg/L Mn |
|---|---|---|---|
| *B. animalis* subsp. *animalis* | 1024 | 956 | 952 |
| *B. breve* | 1088 | | 1068 |
| BB12 | 904 | 1004 | 1052 |

### Example 2: Supplementation of reconstituted milk media with MnSO₄ together with cysteine.

Reconstituted milk was prepared and fermented using *B. animalis* subsp. *animalis, B. breve* or BB12 as provided above. As in Example 1 it was observed that the addition of MnSO₄ together with cysteine to the reconstituted milk reduced the time to reach pH 5.5 for *B*. *animalis* subsp. *animalis* & *B. breve* but not for BB12.

**Table 2: Addition of MnSO₄ together with cysteine reduces time to pH 5.5 for selected Bifidobacteria**

| Strain | Dairy Mix | Time to pH 5.5 |
|---|---|---|
| *B. animalis* subsp. *animalis* | Reconstituted milk | 1024 |
| | Reconstituted milk + 50mg/L Mn + 15mg/L Cysteine | 988 |
| | Reconstituted milk + 15mg/L Mn + 50mg/L Cysteine | 736 |
| *B. breve* | Reconstituted milk | 1088 |
| | Reconstituted milk + 50mg/L Mn + 15mg/L Cysteine | 956 |
| | Reconstituted milk + 15mg/L Mn + 50mg/L Cysteine | 760 |
| BB12 | Reconstituted milk | 904 |
| | Reconstituted milk + 50mg/L Mn + 15mg/L Cysteine | 1372 |
| | Reconstituted milk + 15mg/L Mn + 50mg/L Cysteine | 924 |

### Example 3: Supplementation of reconstituted milk media with yeast.

The addition of yeast extract further reduced the time to pH 5.5 for *B*. *animalis animalis,* both when used in the presence of MnSO₄ together with cysteine, and also when added as the sole supplement to reconstituted milk.

| Strain | Dairy Mix | Time to pH 5.5 |
|---|---|---|
| *B. animalis* subsp. *animalis* | Reconstituted milk | 1024 |
| | Reconstituted milk + 0.25 g/L yeast extract | 746 |
| | Reconstituted milk + 50mg/L Mn + 15mg/L Cysteine + 0.25 g/L yeast extract | 596 |
| | Reconstituted milk + 15mg/L Mn + 50mg/L Cysteine + 0.25 g/L yeast extract | 524 |

## Claims

1. A process for the preparation of a fermented milk product comprising the following steps:
i) providing a mixture comprising:
a) milk
b) at least one bacteria species, comprising at least one *Bifidobacteria* selected from the group consisting of *Bifidobacterium animalis* subsp. *animalis* and *Bifidobacterium breve,*
c) a manganese compound
ii) fermentation of said mixture to provide a fermented milk product,
wherein the amount of manganese compound in said mixture i) is at least 5 mg/l.

2. A process according to claim 1 wherein the amount of manganese compound in said mixture i) is between 5-100 mg/l, preferably between 5-65 mg/l.

3. A process according to claim 1 or 2, wherein the manganese compound is a manganese salt.

4. A process according to claim 3, wherein the manganese salt is selected from the group consisting of MnCI2 and MnSO₄.

5. A process according to any of the preceding claims, wherein the amount of *Bifidobacterium* in said mixture i) is 10⁶-10⁸ cfu/g.

6. A product obtainable by the process according to any one of the preceding claims.

7. A product according to claim 6, wherein said product comprises at least 10⁶ cfu/g *Bifidobacteria.*

8. Use of a manganese compound in the culture of *Bifidobacterium animalis* subsp. *animalis* and/or *Bifidobacterium breve,*
wherein the amount of manganese compound is at least 50 mg/l.

9. An inoculum for the preparation of fermented milk products comprising
i) at least one bacteria species, comprising at least one *Bifidobacteria* selected from the group consisting of *Bifidobacterium animalis* subsp. *animalis* and *Bifidobacterium breve* and,
ii) a manganese compound in an amount of at least 0.1% w/w
wherein i) and ii) are provided in the form of a mixture or as a kit of parts.

10. An inoculum according to any one of the preceding claims, wherein the inoculum or bacteria thereof are fresh, frozen or lyophilized.

11. An inoculum according to claim 10, wherein the manganese compound is a manganese salt.

12. An inoculum according to claim 11, wherein the manganese salt is selected from the group consisting of MnCI2 and MnSO₄.

13. A process, product or inoculum according to any one of the preceding claims, wherein the mixture or inoculum further comprises cysteine and/or yeast extract.

14. A process, product, use or inoculum according to any one of the preceding claims, wherein the at least one bacteria further comprises *Lactobacillus bulgaricus* and/or *Streptococcus thermophilus.*

15. A process, product, use or inoculum according to any one of the preceding claims, wherein said *Bifidobacteria* is *Bifidobacterium animalis* subsp. *animalis* strain CNCM I-4602 and/or *Bifidobacterium breve* strain CNCM 1-4321.

## Patentansprüche

1. Prozess zur Herstellung eines fermentierten Milchprodukts, die folgenden Schritte umfassend:
i) Bereitstellen einer Mischung, umfassend:
a) Milch
b) mindestens eine Bakterienspezies, umfassend mindestens ein *Bifidobacteria* ausgewählt aus der Gruppe, bestehend aus *Bifidobacterium animalis* subsp. *animalis* und *Bifidobacterium breve,*
c) eine Manganverbindung
ii) Fermentieren der Mischung, um ein fermentiertes Milchprodukt bereitzustellen,
wobei die Menge an Manganverbindung in der Mischung i) mindestens 5 mg/l beträgt.

2. Prozess nach Anspruch 1, wobei die Menge an Manganverbindung in der Mischung i) zwischen 5-100 mg/l, bevorzugt zwischen 5-65 mg/l beträgt.

3. Prozess nach Anspruch 1 oder 2, wobei die Manganverbindung ein Mangansalz ist.

4. Prozess nach Anspruch 3, wobei das Mangansalz ausgewählt ist aus der Gruppe, bestehend aus MnCl2 und MnSO₄.

5. Prozess nach einem der vorstehenden Ansprüche, wobei die Menge an *Bifidobacterium* in der Mischung i) 10⁶-10⁸ KbE/g beträgt.

6. Produkt, das durch den Prozess nach einem der vorstehenden Ansprüche erhältlich ist.

7. Produkt nach Anspruch 6, wobei das Produkt mindestens 10⁶ KbE/g *Bifidobacteria* umfasst.

8. Verwendung einer Manganverbindung in der Kultur von *Bifidobacterium animalis* subsp. *animalis* und/oder *Bifidobacterium breve,*
wobei die Menge an Manganverbindung mindestens 50 mg/l beträgt.

9. Inokulum zur Herstellung von fermentierten Milchprodukten, umfassend
i) mindestens eine Bakterienspezies, umfassend mindestens ein *Bifidobacteria* ausgewählt aus der Gruppe, bestehend aus *Bifidobacterium animalis* subsp. *animalis* und *Bifidobacterium breve* und,
ii) eine Manganverbindung in einer Menge von mindestens 0,1 % w/w,
wobei i) und ii) in der Form einer Mischung oder als Teilesatz bereitgestellt sind.

10. Inokulum nach einem der vorstehenden Ansprüche, wobei das Inokulum oder die Bakterien davon frisch, gefroren oder lyophilisiert sind.

11. Inokulum nach Anspruch 10, wobei die Manganverbindung ein Mangansalz ist.

12. Inokulum nach Anspruch 11, wobei das Mangansalz ausgewählt ist aus der Gruppe, bestehend aus MnCl2 und MnSO₄.

13. Prozess, Produkt oder Inokulum nach einem der vorstehenden Ansprüche, wobei die Mischung oder das Inokulum weiter Cystein und/oder Hefeextrakt umfasst.

14. Prozess, Produkt, Verwendung oder Inokulum nach einem der vorstehenden Ansprüche, wobei die mindestens einen Bakterien weiter *Lactobacillus bulgaricus* und/oder *Streptococcus thermophilus* umfassen.

15. Prozess, Produkt, Verwendung oder Inokulum nach einem der vorstehenden Ansprüche, wobei das *Bifidobacteria Bifidobacterium animalis* subsp. *animalis* Stamm CNCM I-4602 und/oder *Bifidobacterium breve* Stamm CNCM 1-4321 ist.

## Revendications

1. Procédé de préparation d'un produit laitier fermenté comprenant les étapes suivantes :
i) la fourniture d'un mélange comprenant :
a) du lait
b) au moins une espèce bactérienne, comprenant au moins une *Bifidobactérie* choisie dans le groupe constitué de *Bifidobacterium animalis* subsp. *animalis* et *Bifidobacterium breve,*
c) un composé de manganèse
ii) la fermentation dudit mélange pour fournir un produit laitier fermenté,
dans lequel la quantité de composé de manganèse dans ledit mélange i) est d'au moins 5 mg/l.

2. Procédé selon la revendication 1, dans lequel la quantité de composé de manganèse dans ledit mélange i) est comprise entre 5 et 100 mg/l, de préférence entre 5 et 65 mg/l.

3. Procédé selon la revendication 1 ou 2, dans lequel le composé de manganèse est un sel de manganèse.

4. Procédé selon la revendication 3, dans lequel le sel de manganèse est choisi dans le groupe constitué de MnCl2 et MnSO₄.

5. Procédé selon l'une quelconque des revendications précédentes, dans lequel la quantité de *Bifidobacterium* dans ledit mélange i) est de 10⁶ à 10⁸ cfu/g.

6. Produit susceptible d'être obtenu par le procédé selon l'une quelconque des revendications précédentes.

7. Produit selon la revendication 6, dans lequel ledit produit comprend au moins 10⁶ cfu/g de *Bifidobactérie.*

8. Utilisation d'un composé de manganèse dans la culture de *Bifidobacterium animalis* subsp. *animalis* et/ou *Bifidobacterium breve,*
dans laquelle la quantité de composé de manganèse est d'au moins 50 mg/l.

9. Inoculum pour la préparation de produits laitiers fermentés comprenant
i) au moins une espèce bactérienne, comprenant au moins une *Bifidobactérie* choisie dans le groupe constitué de *Bifidobacterium animalis* subsp. *animalis* et *Bifidobacterium breve* et,
ii) un composé de manganèse en une quantité d'au moins 0,1 % p/p,
dans lequel i) et ii) sont fournis sous la forme d'un mélange ou d'un kit.

10. Inoculum selon l'une quelconque des revendications précédentes, dans lequel l'inoculum ou les bactéries de celui-ci sont frais, congelés ou lyophilisés.

11. Inoculum selon revendication 10, dans lequel le composé de manganèse est un sel de manganèse.

12. Inoculum selon revendication 11, dans lequel le sel de manganèse est choisi dans le groupe constitué de MnCl₂ et MnSO₄.

13. Procédé, produit ou inoculum selon l'une quelconque des revendications précédentes, dans lequel le mélange ou l'inoculum comprend en outre de la cystéine et/ou de l'extrait de levure.

14. Procédé, produit, utilisation ou inoculum selon l'une quelconque des revendications précédentes, dans lequel ladite au moins une bactérie comprend en outre *Lactobacillus bulgaricus* et/ou *Streptococcus thermophilus.*

15. Procédé, produit, utilisation ou inoculum selon l'une quelconque des revendications précédentes, dans lequel ladite *Bifidobacteria* est une souche de *Bifidobacterium animalis* subsp. *animalis* CNCM I-4602 et/ou une souche de *Bifidobacterium breve* CNCM 1-4321.
